(19) Europäisches Patentamt
European Patent Office
Office européen des brevets

(11) **EP 4 810 006 A1**

(12) **EUROPEAN PATENT APPLICATION**

(43) Date of publication:
**23.09.2026 Bulletin 2026/39**

(21) Application number: **26166439.5**

(22) Date of filing: **20.03.2026**

(51) International Patent Classification (IPC):
***A61K 8/34*** $^{(2006.01)}$ ***A61K 8/73*** $^{(2006.01)}$
***A61K 8/9789*** $^{(2017.01)}$ ***A61Q 19/00*** $^{(2006.01)}$

(52) Cooperative Patent Classification (CPC):
**A61K 8/9789; A61K 8/345; A61K 8/735; A61Q 19/00**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH LA MA MD TN**

(30) Priority: **21.03.2025 FI 20255240**

(71) Applicant: **Lumene Oy**
**02780 Espoo (FI)**

(72) Inventors:
- **MAVON, Alain**
  **02780 Espoo (FI)**
- **PESONEN, Terhi**
  **02780 Espoo (FI)**
- **MÄKINEN, Päivi**
  **02780 Espoo (FI)**
- **PALMUJOKI, Ingela**
  **02780 Espoo (FI)**
- **SAHRAMO, Elina**
  **02780 Espoo (FI)**

(74) Representative: **Laine IP Oy**
**Porkkalankatu 24**
**00180 Helsinki (FI)**

Remarks:
CORRECT PARTS INCLUDED UNDER RULE 56a(4) EPC

# (54) COSMETIC COMPOSITIONS FOR INCREASING SKIN HYDRATION AND SKIN BARRIER FUNCTION

(57) The present invention relates to cosmetic formulations comprising hyaluronic acids, xylitol, birch leaf extract and/or birch bark extract, and optional ingredients commonly used in cosmetic formulations. The invention also relates to non-therapeutic, cosmetic use of said formulations for increasing skin hydration and/or skin barrier function. Further, the invention relates to a non-therapeutic, cosmetic use of said formulations for upregulating skin hydration markers selected from loricrin, trichohyalin, repetin, comeodesmosin and filaggrin, preferably loricrin and trichohyalin.

gene expression % of indivudual ingredients and in combination
( at 0,37% - & 0,041%) % of expression - 100 is the reference /control
500
450
400
350
300
250
200
150
100
HA
BL
X
HABLX
LOR
TCHH
CDSN
LOR
TCHH
FLG

Fig. 1



Processed by Luminess, 75001 PARIS (FR)

## Description

### FIELD

The present invention relates to cosmetic formulations comprising hyaluronic acids, xylitol, birch leaf extract and/or birch bark extract, as well as optional ingredients commonly used in cosmetic formulations. The invention also relates to non-therapeutic, cosmetic use of said formulations for increasing skin hydration and/or skin barrier function. Further, the invention relates to a non-therapeutic, cosmetic use of said formulations or the combination of hyaluronic acids, xylitol, birch leaf extract and/or birch bark extract for upregulating skin hydration markers selected from loricrin, trichohyalin, repetin, corneodesmosin and filaggrin, preferably loricrin and/or trichohyalin, and to a method for moisturizing skin and for strengthening skin barrier function by upregulating the expression of skin hydration biomarkers selected from loricrin, trichohyalin, repetin, corneodesmosin and filaggrin.

### BACKGROUND

The human skin protects body from external aggressors as well as from water loss. The barrier function of the skin is mainly located in the stratum corneum. Defects in the stratum corneum permeability barrier cause varieties of skin concerns, including dry and dehydrated skin. Mechanical resilience of the stratum corneum is linked to cornified cell envelopes. Cornification in turn largely depends on the properties of loricrin, which is a major component in cell envelopes (Ishitsuka and Roop, 2020). In fact, loricrin represents over 70% of the cornified envelope in the epidermis. It plays a crucial role in reinforcing the skin barrier, providing mechanical strength, flexibility, and water retention. Loricrin interacts with natural moisturizing factors (NMFs) and lipids, helping to regulate hydration and reduce transepidermal water loss (TEWL). Studies have shown that lower loricrin levels are associated with reduced skin hydration and weakened barrier function, leading to dryness and increased susceptibility to unbalanced skin. Thus loricrin is a terminally differentiating structural protein that contributes to the protective barrier function of the stratum corneum.

The other above mentioned skin hydration biomarkers (epidermal proteins) are also known to be beneficial to hydration of skin or to mechanical strengthening of the skin. Among these epidermal proteins, trichohyalin is of particular interest as it is a structural protein primarily found in the inner root sheath (IRS) of hair follicles and other toughened epithelial tissues. Its main function is to enhance the mechanical strength of these structures through the formation of the Cornified Cell Envelope (CE). Trichohyalin serves as a major reinforcement cross-bridging protein for the cell envelope barrier structure of the IRS. By cross-linking with other CE proteins, such as involucrin, envoplakin, repetin, and desmoplakin, trichohyalin contributes to the formation of a robust, insoluble barrier that protects epithelial cells from mechanical damage and provides rigidity and mechanical strength to tissues like the hair follicle's inner root sheath (Steinert et al, 2003).

It would thus be beneficial in terms of cosmetic skin care to stimulate or upregulate the expression of the above discussed hydration markers and thus to reduce or minimize moisture loss, hydrate the skin and strengthen the barrier function of the skin, thus improving skin appearance. Furthermore, upregulating said markers could be beneficial also in providing hair care products with beneficial hair care properties.

### SUMMARY OF THE INVENTION

The invention is defined by the features of the independent claims. Some specific embodiments are defined in the dependent claims.

The present invention is based on the finding that a combination of hyaluronic acids and Nordic xylitol together with Nordic birch leaf and/or birch bark extract has an ability to promote production of skin hydration markers, such as loricrin, and therefore enhance and strengthen barrier function (better barrier cohesion, strengthening effect), minimize water loss and deliver moisturizing and skin-protective and thus skin appearance improving effects on the skin. The effect on skin hydration markers has been demonstrated by in vitro studies. Further, the moisturizing and skin-protective effects have been demonstrated by in vivo studies with healthy volunteers.

According to a first aspect of the present invention, there is provided a cosmetic composition comprising, as cosmetically active agents, hyaluronic acids, Nordic xylitol and Nordic birch leaf extract and/or Nordic birch bark extract.

According to a second aspect of the present invention, there is provided non-therapeutic, cosmetic use of a composition according to the first aspect of the invention for increasing skin hydration and/or skin barrier function on skin of the face, body or the scalp of a user.

According to a further aspect, there is provided non-therapeutic, cosmetic use of a composition according to the invention for upregulating expression of skin hydration markers selected from loricrin, trichohyalin, repetin, corneodesmosin and filaggrin.

The invention also provides combined non-therapeutic cosmetic use of hyaluronic acids and Nordic xylitol,

together with Nordic birch leaf extract and/or Nordic birch bark extract in a cosmetic composition for increasing the skin moisturizing effect and/or the skin barrier strengthening effect of the cosmetic composition on human skin.

According to a still further aspect of the present invention, there is provided a method for moisturizing skin and for strengthening skin barrier function by upregulating expression of skin hydration markers selected from loricrin, tricohyalin, repetin, corneodesmosin and filaggrin, the method comprising including hyaluronic acids, Nordic xylitol, and Nordic birch leaf extract and/or Nordic birch bark extract into a cosmetic composition and applying the cosmetic composition on the skin of the face, body or the scalp of a user.

Considerable advantages are obtained by the invention. First, a combination of hyaluronic acids and Nordic xylitol together with Nordic birch leaf and/or birch bark extract has a synergistic effect on the expression of certain skin hydration markers linked to skin hydrating and moisturizing properties, to skin barrier properties and to beneficial hair care properties.

Second, a combination of Nordic birch leaf extract with hyaluronic acids and Nordic xylitol is particularly associated to beneficial skin applications, where the combination upregulates expression of loricrin, thus decreasing stratum corneum permeability to improve skin barrier function and thus skin appearance, in addition to providing skin moisturizing effects.

In addition, a combination of Nordic birch bark extract with hyaluronic acids and Nordic xylitol is particularly associated to beneficial applications for hair and scalp care, where the combination upregulates expression of trichohyalin, thus mechanically strengthening the hair follicles.

Further features and advantages of the present technology will appear from the following description of some embodiments.

BRIEF DESCRIPTION OF THE DRAWINGS

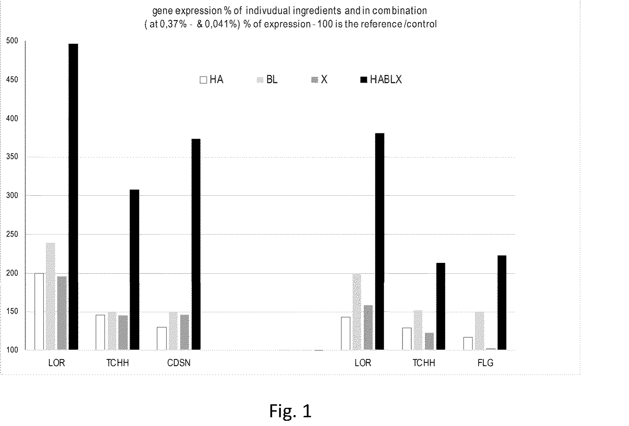

FIGURE 1 illustrates summary of the results obtained when the samples (combination of ingredients vs individual ingredients) were tested for stimulation or upregulation of genes of different hydration markers on human keratinocytes. Samples: HA = blend of three hyaluronic acids, BL = birch leaf extract, X = xylitol, HABLX = triple hyaluronic acid blend + birch leaf extract + xylitol. Hydration markers: LOR = loricrin, TCHH = trichohyalin, RPTN = repetin, CDSN = corneodesmosin, FLG = filaggrin.

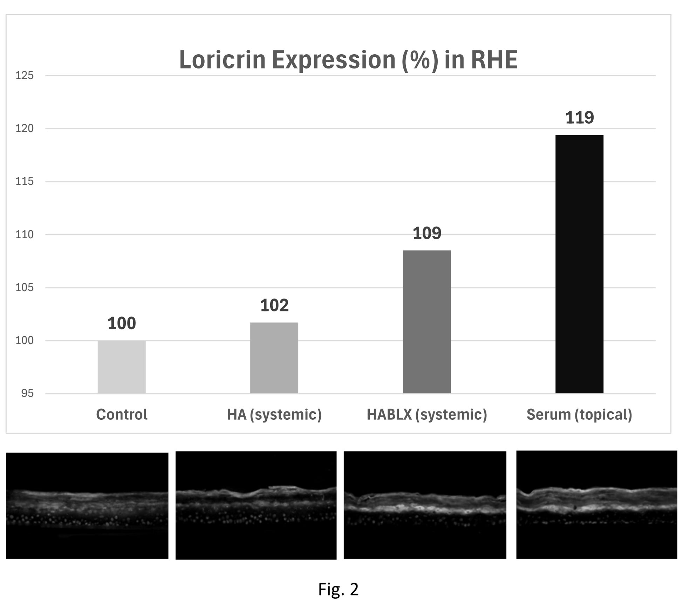

FIGURE 2 illustrates loricrin expression on human reconstituted epidermis, showing loricrin immunostaining and the corresponding fluorescence quantification (%) normalised versus the control, with 1. systemic application of HA vs HABLX and 2. topical application of a serum formulation containing the HABLX technology blend (Composition example 2).

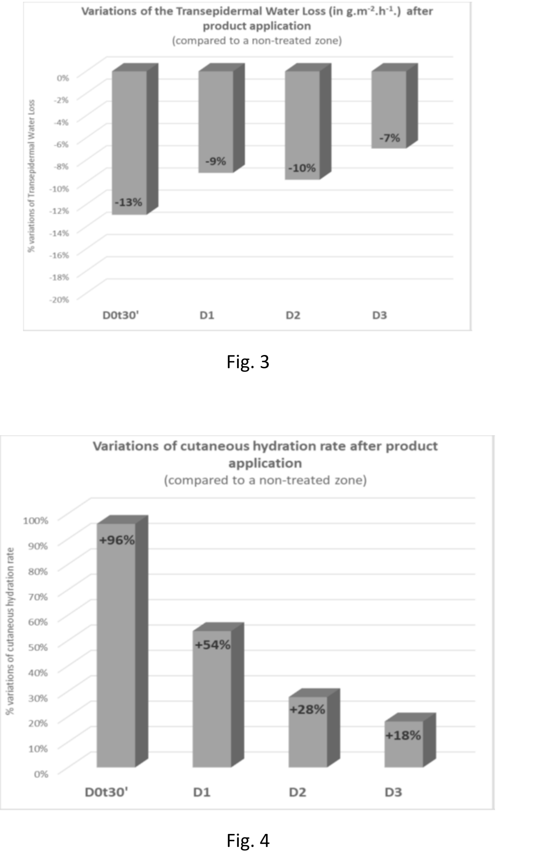

FIGURE 3 illustrates variations of the transepidermal water loss (in $g \cdot m^{-2} \cdot h^{-1}$) after application of a product (a cream according to Composition Example 1) on the skin of healthy volunteers, compared to a non-treated zone. Time: D0t30'= after 30 minutes, D1 = day 1, D2 = day 2, D3 = day 3.

FIGURE 4 illustrates variations of cutaneous hydration rates after application of a product (a cream according to Composition Example 1) on the skin of healthy volunteers, compared to a non-treated zone. Time: D0t30'= after 30 minutes, D1 = day 1, D2 = day 2, D3 = day 3.

EMBODIMENTS

DEFINITIONS

In the present context, the term "Nordic" in connection with ingredients, such as birch extracts, indicates that the plants from which the ingredients are obtained are grown in the Nordic area. The Nordic area refers to a geographical region in Northern Europe and the North Atlantic. Preferably, the "Nordic ingredients" referred herein include ingredients obtained from plants growing in the Nordic countries, which by definition include Denmark, Finland, Iceland, Norway, and Sweden, as well as Greenland, the Faroe Islands, the Åland islands and Svalbard archipelagos.

Within this disclosure, "cosmetically active agents" refer to cosmetically acceptable substances that work to address specific skin, scalp or hair concerns and have an effect in the improvement of appearance of the human user.

The present invention is based on the finding that a combination of certain cosmetic ingredients, in particular a combination of hyaluronic acids, Nordic xylitol and Nordic birch leaf extract and/or Nordic birch bark extract, provides a synergistic cosmetic effect when used in combination, compared to their use as individual cosmetic ingredients. In

particular, the cosmetic compositions of the present kind reinforce and strengthen barrier function of the skin, thus improving skin appearance, reduce or minimize moisture loss, moisturize and hydrate the skin or the scalp, and may also mechanically strengthen hair follicles.

The present cosmetic composition comprises hyaluronic acids, Nordic xylitol and Nordic birch leaf extract and/or Nordic birch bark extract as cosmetically active agents. The cosmetic composition may also comprise also other cosmetically acceptable substances typically used in cosmetic compositions, such as thickeners, bulking agents, UV filters, emulsifying agents, surfactants, emollients, preservatives, antimicrobial agents, colorants, film forming agents, flavouring agents, foam boosting agents, parfum, oils, waxes and fats, alcohols, and water.

In one embodiment, the cosmetic composition comprises hyaluronic acids, Nordic xylitol, and Nordic birch leaf extract as active agents. The cosmetic composition comprising hyaluronic acids, Nordic xylitol, and Nordic birch leaf extract is preferably a skin care composition. In some embodiments the cosmetic composition comprises hyaluronic acids, Nordic xylitol and Nordic birch leaf extract as the sole active agents.

In one embodiment, the cosmetic composition according to the invention comprises hyaluronic acids, Nordic xylitol, and Nordic birch bark extract as active agents. The cosmetic composition comprising hyaluronic acids, Nordic xylitol, and Nordic birch leaf extract is preferably a hair and/or scalp care composition. In some embodiments the cosmetic composition comprises hyaluronic acids, Nordic xylitol and Nordic birch bark extract as the sole active agents.

## Hyaluronic acids

The combination formulation according to the invention comprises hyaluronic acids. Hyaluronic acids are well known and commonly used hydrating ingredients used in cosmetic products.

In the present invention hyaluronic acids are typically used as a blend of hyaluronic acids having different average molecular weights, preferably as a blend of at least three hyaluronic acids having different average molecular weights. More preferably, a blend of large, medium and small molecular weight hyaluronic acids is used, such as hyaluronic acids having an average molecular weight of about 1000 kDa or more, hyaluronic acids having an average molecular weight of approximately 300 kDa, and hyaluronic acids having an average molecular weight of about 50 kDa. In some embodiments, the blend of hyaluronic acids may comprise or consist of at least one hyaluronic acid having an average molecular weight above 3000 kDa, at least one hyaluronic acid having an average molecular weight of 200-400 kDa, and at least one hyaluronic acid having an average molecular weight of 37-50 kDa. The concentration of hyaluronic acids in a cosmetic composition of the present invention may vary but is typically 0.001%- 50% by weight of the cosmetic composition.

All hyaluronic acids provide a moisturizing effect. More specifically, hyaluronic acids having a higher molecular weight provide an immediate moisturizing effect, while hyaluronic acids having a smaller molecular weight, such as 200-400 kDa, provide a long-term deep moisturizing effect and increase elasticity of the skin. Further, hyaluronic acids having an ultrasmall molecular weight, such as 37-50 kDa, smoothen the skin and fill in wrinkles. Examples of suitable hyaluronic acids or salt thereof include but are not limited to sodium hyaluronate, hydrolysed hyaluronic acid, and sodium hyaluronate.

## Xylitol

Xylitol is typically used in cosmetic products as a moisturizing agent. In a preferred embodiment, the xylitol used in the present invention is Nordic xylitol, typically obtained by processing Nordic oat hulls, Nordic birch cellulose or any other Nordic naturally derived source, preferably Nordic oat hulls or Nordic birch cellulose. Recently, oat hulls, which are derived as a side stream from oat milling process, have been used in the production of Nordic oat xylitol. In brief, oat hulls are hydrolysed, the obtained hydrolysate is separated to a solid fraction and to a carbohydrate containing liquid fraction, which is rich in xylose and may be used in the production of xylitol. The Nordic xylitol extracted from birch bark also known as "wood sugar" is extracted using hydrolysis, once the waste products have been removed. It is then hydrogenated in order to crystallize it.

The concentration of Nordic xylitol in a cosmetic composition according to the present invention may vary but is typically 0,001%-50% by weight of the cosmetic composition.

## Birch leaf extract and birch bark extract

The birch leaf extract used in the present invention is typically obtained from fresh or dried birch leaves by a method comprising the steps of extracting the leaves with a solvent, preferably with circulating glycerin:water, with water:butyleneglycol or with pentyleneglycol, propanediol or ethanol, more preferably with circulating glycerine:water at room temperature to obtain the birch leaf extract. Optionally, the obtained birch leaf extract may be dried, typically by spray-drying, to obtain birch leaf powder. In embodiments, the birch leaf extract is typically a water/glycerine extract of Nordic birch leaves.

The birch bark extract used in the present invention is typically obtained from Nordic birch bark by a method comprising the steps of extracting the bark with a solvent, typically with water/glycerine, to obtain the birch bark extract. Optionally, the obtained Nordic birch bark extract may be dried, typically by spray-drying, to obtain birch bark powder. In embodiments, the birch bark extract is typically a water/glycerine extract of Nordic birch bark.

The birch leaf extract and the birch bark extract are derived from Nordic birch leaves or Nordic birch bark, respectively, in particular from leaves or bark from Nordic birches *Betula pendula, Betula alba* and/or *Betula pubescens.*

Nordic birch extracts are naturally enriched with higher concentrations of potential active substances, such as polyphenols, as compared for example to a French equivalent extract. Polyphenols are known substances providing several benefits for skin and hair health (Mang Sun et al, 2022). There may also be some natural variation within the Nordic area depending on the place of growth of the birch, the growth stage and surrounding conditions. By way of an example, the concentrations of polyphenols, sugars and monosaccharides in a typical Nordic birch leaf extract are shown in the below table, compared to a birch leaf extract derived from French birch leaves by the same extraction method.

| Substance (HPLC analysis) | Finnish BL extract | French BL extract |
|---|---|---|
| Polyphenols (GT0311) | 0.292 g/L | 0.138 g/L |
| Polyphenols Folin (GT269) | 0.375 g/L | 0.226 g/L |
| Total sugars (GT035) | 2.78 g/L | 1.63 g/L |
| Total monosaccharides (GT045 | 2.54 g/L | 1.57 g/L |

The cosmetic composition according to the invention contains a combination of hyaluronic acids, birch leaf extract and/or birch bark extract, and xylitol. The ratio of the components in the combination may vary within wide limits. In some embodiments, the weight ratio between 1) hyaluronic acids, 2) birch leaf extract and/or birch bark extract, and 3) xylitol is approximately 1:1:1. However, other ratios are also possible and within the scope of the invention.

Typically, the combined concentration of hyaluronic acids, xylitol, and birch leaf extract and/or birch bark extract in the cosmetic composition is 0.001-50%, such as 0.003-50%, preferably 0.05-10%, based on the weight of the cosmetic composition. The composition may further comprise conventional cosmetically acceptable substances.

**Hydration markers**

As stated above, the cosmetic composition according to the invention increases the expression of at least one or more hydration markers selected from the group consisting of loricrin, trichohyalin, repetin, corneodesmosin and filaggrin. In particular, the combination formulation according to the present invention has a synergistic effect on the expression of skin hydration markers (epidermal genes) linked to skin hydrating and moisturizing properties. The combination of hyaluronic acids and Nordic xylitol together with Nordic birch leaf and/or birch bark extract upregulates skin hydration markers selected from loricrin, tricohyalin, repetin, corneodesmosin, filaggrin. In particular, the combination formulation of the invention upregulates (stimulates) loricrin and trichohyalin.

**Loricrin**

Loricrin (LOR) is a cysteine-rich cornified cell envelope (CE) protein that is critical in establishing the biochemical properties of the epidermis. Loricrin is a terminally differentiating structural protein comprising more than 70% of the cornified envelope. It plays a crucial role in reinforcing the skin barrier, providing mechanical strength, flexibility, and water retention. Loricrin interacts with natural moisturizing factors (NMFs) and lipids, helping to regulate hydration and reduce transepidermal water loss (TEWL). Studies have shown that lower loricrin levels are associated with reduced skin hydration and weakened barrier function, leading to dryness and increased susceptibility to unbalanced skin (Nithya et al, 2015).

**Trichohyalin**

Trichohyalin is expressed in specialized epithelia that are unusually mechanically strong, such as the inner root sheath cells of the hair follicle. It has been suggested that trichohyalin serves as an interfilamentous matrix protein by becoming cross-linked both to itself and to the head and tail end domains of the inner root sheath keratin intermediate filament chains (Steinert et al, 2003). Further, trichohyalin serves as a cross-bridging reinforcement protein of the cornified cell envelope of the inner root sheath cells by becoming crosslinked to several known or novel barrier proteins, including involucrin, small proline-rich proteins, repetin, and epiplakin. It also coordinates linkage between the keratin filaments and

cell envelope to form a seamless continuum (Steinert et al, 2003).

The above shows that trichohyalin is a multi-functional cross-bridging protein that functions in the inner root sheath and perhaps in other specialized epithelial tissues by conferring to and coordinating mechanical strength between their peripheral cell envelope barrier structures and their cytoplasmic keratin filament networks. Increasing expression of trichocyalin in cells, in particular in the inner root sheath cells of the hair follicle, would thus be advantageous for cosmetic treatment of hair and scalp. Since the cosmetic composition of the present invention, especially the cosmetic composition comprising hyaluronic acids, oat xylitol and birch bark extract, was confirmed to have the ability to increase the expression of trichohyalin, the cosmetic composition of the present invention may be used as a cosmetic hair care composition, where the increased expression of trichohyalin mechanically strengthens the hair follicle.

**Corneodesmosin, filaggrin and repetin**

Corneodesmosomes are main intercellular adhesive structures in the stratum corneum. They are transformed from desmosomes at the most superficial layer of the stratum granulosum of the epidermis (Ishida-Yamamoto and Igawa, 2015).

Filaggrin is a structural protein that is fundamental in the development and maintenance of the skin barrier having a fundamental role in terminal epidermal differentiation and for its implication in some of the most common dermatological diseases, such as atopic dermatitis and ichthyosis vulgaris (Armengot-Carbo et al, 2015).

Repetin is a member of the "fused" gene family and is localized in the epidermal differentiation complex on chromosome 1q21. The "fused" gene family comprises profilaggrin, trichohyalin, repetin, hornerin, the profilaggrin-related protein and a protein encoded by c1orf10. Functionally, these proteins are associated with keratin intermediate filaments and partially crosslinked to the cell envelope (CE) (Huber et al, 2005).

Among the above disclosed hydration markers, trichohyalin is a marker expressed particularly in scalp where its expression mechanically strengthens the hair follicle. Loricrin, corneodesmosin, filaggrin and repetin belong to hydration markers expressed mainly on skin. In some embodiments, typically in cosmetic skin care applications, the compositions disclosed herein upregulate in particular the expression of loricrin, preferably loricrin and trichohyalin.

In one embodiment, the cosmetic composition according to the invention comprises active agents which consist or consist essentially of hyaluronic acids, xylitol, and Nordic birch leaf extract. The above composition increases the expression of skin hydration markers and is preferably a skin care composition. In particular, the cosmetic composition comprising active agents, which consist or consist essentially of hyaluronic acids, xylitol, and Nordic birch leaf extract, increases the expression of loricrin, thus providing a moisturizing and skin barrier strengthening effect on the skin.

Typically, the combined concentration of hyaluronic acids, birch leaf extract and xylitol in the cosmetic composition is 0.05-10 % by weight of the composition.

In one embodiment, the cosmetic composition is a hair care composition comprising active agents which consist of or consist essentially of hyaluronic acids, xylitol and Nordic birch bark extract. The above composition increases in particular the expression of trichohyalin. The cosmetic composition comprising active agents, which consist or consist essentially of hyaluronic acids, xylitol, and Nordic birch bark extract, increases the expression of trichohyalin, thus providing a hair follicle strengthening effect on the scalp skin. Typically, the combined concentration of hyaluronic acids, birch bark extract and xylitol in the cosmetic hair care composition is 0.05-10 % by weight of the composition.

Cosmetic compositions according to the invention include but are not limited to skin care creams, lotions or gels, tonic cosmetic waters, serums, skin oils (bi-phase or tri-phase), lip care products (sticks, creams, gels), skin cleansing creams, lotions or gels, foundations, concealers, self-tan lotions or creams, shampoos (liquids, creams, pastes), hair conditioners, hair/scalp care lotions, and combined shampoo + conditioner products.

The invention also relates to non-therapeutic, cosmetic use of the compositions of the invention for increasing skin hydration and/or skin barrier function on skin of the face, body or the scalp of a user. In particular, the invention also relates to non-therapeutic, cosmetic use of the compositions according to the invention for upregulating expression of skin hydration markers selected from loricrin, trichohyalin, repetin, corneodesmosin and filaggrin, in particular loricrin and/or trichohyalin.

Moreover, the invention provides a combined non-therapeutic cosmetic use of hyaluronic acids, Nordic xylitol, and Nordic birch leaf extract and/or Nordic birch bark extract in a cosmetic composition for increasing the skin moisturizing effect and/or the skin barrier strengthening effect of the cosmetic composition on human skin. More particularly, the combined use upregulates expression of skin hydration markers selected from loricrin, trichohyalin, repetin, corneodesmosin and filaggrin, in particular loricrin and trichohyalin.

Correspondingly, the present invention also provides a method for moisturizing skin and for strengthening skin barrier function by upregulating expression of skin hydration markers selected from loricrin, trichohyalin, repetin, corneodesmosin and filaggrin, the method comprising including hyaluronic acids, Nordic xylitol, and Nordic birch leaf extract and/or Nordic birch bark extract into a cosmetic composition and applying the cosmetic composition on the skin of the face, body or the scalp of a user.

It is to be understood that the embodiments of the invention disclosed are not limited to the particular structures, process steps, or materials disclosed herein, but are extended to equivalents thereof as would be recognized by those ordinarily skilled in the relevant arts. It should also be understood that terminology employed herein is used for the purpose of describing particular embodiments only and is not intended to be limiting.

Reference throughout this specification to one embodiment or an embodiment means that a particular feature, structure, or characteristic described in connection with the embodiment is included in at least one embodiment of the present invention. Thus, appearances of the phrases "in one embodiment" or "in an embodiment" in various places throughout this specification are not necessarily all referring to the same embodiment. Where reference is made to a numerical value using a term such as, for example, about or substantially, the exact numerical value is also disclosed.

As used herein, a plurality of items, structural elements, compositional elements, and/or materials may be presented in a common list for convenience. However, these lists should be construed as though each member of the list is individually identified as a separate and unique member. Thus, no individual member of such list should be construed as a de facto equivalent of any other member of the same list solely based on their presentation in a common group without indications to the contrary. In addition, various embodiments and example of the present invention may be referred to herein along with alternatives for the various components thereof. It is understood that such embodiments, examples, and alternatives are not to be construed as de facto equivalents of one another, but are to be considered as separate and autonomous representations of the present invention.

Furthermore, the described features, structures, or characteristics may be combined in any suitable manner in one or more embodiments. In the following description, numerous specific details are provided, such as examples of lengths, widths, shapes, etc., to provide a thorough understanding of embodiments of the invention. One skilled in the relevant art will recognize, however, that the invention can be practiced without one or more of the specific details, or with other methods, components, materials, etc. In other instances, well-known structures, materials, or operations are not shown or described in detail to avoid obscuring aspects of the invention.

## EXPERIMENTAL

Unless otherwise stated herein or clear from the context, any percentages referred to herein are expressed as percent by weight based on a total weight of the respective composition. Unless otherwise stated, properties that have been experimentally measured or determined herein have been measured or determined at room temperature. Unless otherwise indicated, room temperature is 25°C. Unless otherwise stated, properties that have been experimentally measured or determined herein have been measured or determined at atmospheric pressure.

As used herein, unless otherwise indicated, the term "average molecular weight" refers to a weight average molecular weight (also abbreviated "$M_W$" or "Mw").

### ***In vitro*** **studies: 1. Effect of seven different compounds on gene expression profile of normal human epidermal keratinocytes**

In the present study, the effects of 7 compounds were investigated in normal human epidermal keratinocytes (NHEK) on a specific gene expression profile using RT-qPCR technology. Extracted mRNA was analyzed using a PCR array targeting 32 genes (including 2 housekeeping genes) selected for their importance in barrier function and hydration.

The cell type was Normal human epidermal keratinocytes (NHEK), used at the 3rd passage. Culture conditions were 37°C, 5% $CO_2$, using the following culture medium: Keratinocyte SFM optimized for the assay, supplemented with Epidermal Growth Factor Pituitary Extract. The assay medium was "Keratinocyte SFM optimized for the assay".

Samples tested were marked as follows: HA, X, BB, BL, HAB, HAL, BBX, HABBX, HABLX. The sample codes are explained in Table 1 below.

**Table 1.** Sample codes with explanations

| SAMPLE CODE | EXPLANATION |
|---|---|
| HA | 3x hyaluronic acid blend, large/medium/small molecular weight (HA >3000KD, HA 200-400 KD), HA 37-50KD) |
| X | Xylitol |
| BB | Birch bark extract (glycerin/water) |
| BL | Birch leaf extract (glycerin/water) |
| HAB | Triple hyaluronic acid blend + Birch bark extract |
| HAL | Triple hyaluronic acid blend + Birch leaf extract |

(continued)

| SAMPLE CODE | EXPLANATION |
|---|---|
| BBX | Birch bark extract + Xylitol |
| HABBX | Triple hyaluronic acid blend + Birch bark extract + Xylitol |
| HABLX | Triple hyaluronic acid blend + Birch leaf extract + Xylitol |

Study 1: Various concentrations were tested, based on cytotoxicity

Study 2: Repetition with standardized concentrations

For the concentrations tested in Study 1 and Study 2, see Tables 2 and 3 below.

**Table 2.** Study 1 - Sample concentrations tested, based on cytotoxicity

| Test compound | Aspect/Storage | Intermediate solution | Test concentrations |
|---|---|---|---|
| **HA** Ref. HA | - Gel - Storage: +4°C | 10% in assay medium | 0.111%, 0.333% and 1% |
| **X** Ref. X | - Liquid - Storage: +4°C | 10% in assay medium | 0.367%, 1.1% and 3.3% |
| **BB** Ref. BB | - Liquid - Storage: +4°C | 10% in assay medium | 0.111%, 0.333% and 1% |
| **BL** Ref. BL | - Thick liquid - Storage: +4°C | 10% in assay medium | 0.041%, 0.123% and 0.37% |
| **HAB** Ref. HAB | - Gel - Storage: +4°C | 10% in assay medium | 0.111%, 0.333% and 1% |
| **HAL** Ref. HAL | - Gel - Storage: +4°C | 10% in assay medium | 0.041%, 0.123% and 0.37% |
| **BBX** Ref. BBX | - Liquid - Storage: +4°C | 10% in assay medium | 0.122%, 0.367% and 1.1% |
| **HABBX Ref. HABBX** | - Viscous liquid - Storage: +4°C | 10% in assay medium | 0.111%, 0.333% and 1% |
| **HABLX Ref. HABLX** | - Gel - Storage: +4°C | 10% in assay medium | 0.041%, 0.123% and 0.37% |

**Table 3.** Study 2 - concentrations (repetition of Study 1 with standardized concentrations)

| Test compound | Aspect/Storage | Intermediate solution | Test concentrations |
|---|---|---|---|
| **HA** Ref.HA | - Gel - Storage: +4°C | 1% in assay medium | 0.014%, 0.041% and 0.123% |
| **BL** Ref. BL | - Liquid - Storage: +4°C | 1% in assay medium | 0.014%, 0.041% and 0.123% |
| **X Ref. X** | - Liquid - Storage: +4°C | 1% in assay medium | 0.014%, 0.041% and 0.123% |
| **HABL** Ref. HABL | - Gel - Storage: +4°C | 1% in assay medium | 0.014%, 0.041% and 0.123% |
| **HAX** Ref. | - Gel - Storage: +4°C | 1% in assay medium | 0.014%, 0.041% and 0.123% |
| **BLX** Ref. BLX | - Liquid - Storage: +4°C | 1% in assay medium | 0.014%, 0.041% and 0.123% |
| **HABLX** Ref. HABLX | - Gel - Storage: +4°C | 1% in assay medium | 0.014%, 0.041% and 0.123% |

Keratinocytes were seeded in 24-well plates and cultured for 24 hours in culture medium and in assay medium for a further 24 hours. The medium was then replaced by assay medium containing or not (control) the test compounds or the reference (retinoic acid tested at 10-7 M) and the cells were incubated for 24 hours. All experimental conditions were performed in n=3. At the end of incubation, the cells were washed in phosphate buffered saline (PBS) solution and immediately frozen at 80°C.

The expression of markers was analyzed using RT-qPCR method on total RNA extracted from the cell monolayers of each experimental condition (before RNA extraction, the replicates of the same experimental condition were pooled). The analysis of transcripts was performed in n=2 using a PCR array targeting 32 genes (30 genes + 2 housekeeping genes) selected for their importance in barrier function and hydration. Total RNA was extracted from each sample using TriPure Isolation Reagent® according to the supplier's instructions. The quality of RNA was evaluated using capillary electrophoresis (4200 TapeStation System, Agilent Technologies). The quantity of RNA was evaluated using a spectrophotometer (Synergy H1, BioTek Instruments).

The complementary DNA (cDNA) was synthetized by reverse transcription of total RNA in presence of oligo(dT)

and « Transcriptor Reverse Transcriptase » (Roche). The cDNA quantities were then adjusted before PCR step.

The PCR (Polymerase Chain Reaction) were performed using the « LightCycler® » system (Roche Molecular System Inc.) according to the supplier's instructions. The reaction mix (10 μl final) was prepared as follows: - 2.5 μl of cDNA / primers (forward and reverse) / - reagent mix (Ozyme) containing taq DNA polymerase, SYBR Green I and MgCl2.

Data management:

Raw data were analyzed using Microsoft Excel software. The incorporation of fluorescence in amplified DNA was continuously measured during the PCR cycles. This resulted in a "fluorescence intensity" versus "PCR cycle" plot allowing the evaluation of a relative expression (RE) value for each marker.

The value selected for RE calculations is the "output point" (Ct) of the fluorescence curve. For a considered marker, the highest is the cycle number; the lowest is the mRNA quantity.

The RE value was calculated with the formula: (1/2number of cycles) x 106. The PCR array used in the present study included 2 reference genes (RPS28 and GAPDH). These housekeeping genes were used for data normalization since their expression is constitutive and theoretically stable. Consequently, the level of expression of the target markers was compared to the mean expression level of these 2 markers for all test conditions.

Classification of effects ("treated" conditions versus "control"):

Standardized interpretation was done according to the following table (Table 4):

**Table 4.** Classification of effects

| Relative expression (% of control) | Classification of effects |
|---|---|
| > 300% | Strong stimulation |
| > 200% and < 300% | Stimulation |
| > 150% and < 200% | Slight stimulation, to be confirmed |
| > 50% and < 65% | Moderate inhibition, to be confirmed |
| > 30% and < 50% | Inhibition |
| < 30% | Strong inhibition |

Results:

The treatment of normal human epidermal keratinocytes (NHEK) with the reference Retinoic acid (0.1 μM) for 24 hours resulted in the downregulation of markers involved in terminal keratinocyte differentiation (SPRR1B, SPRR1A and in a lesser extent FLG, TGM1) and in epidermal cohesion (DSG1, DSC1 and in a lesser extent CLDN1). In parallel, retinoic acid induced an upregulation of a marker involved in hyaluronic synthesis (HAS3) and marker CLDN4. These results were expected and validated the assay.

**Table 5** and **Table 6** show an overview of the results of Study 1 and Study 2.

**Table 5**. Overview of the results from Study 1

| mQPA-NHEK-Barrier function and Hydration-32 | Genes | | HA | | | X | | | BB | | | BL | | |
|---|---|---|---|---|---|---|---|---|---|---|---|---|---|---|
| | | | 0.111% | 0.333% | 1% | 0.367% | 1.1% | 3.3% | 0.111% | 0.333% | 1% | 0.041% | 0.123% | 0.37% |
| | Abbreviation | NCBI data base link | % Control Mean HK | % Control Mean HK | % Control Mean HK | % Control Mean HK | % Control Mean HK | % Control Mean HK | % Control Mean HK | % Control Mean HK | % Control Mean HK | % Control Mean HK | % Control Mean HK | % Control Mean HK |
| Keratinocyte differentiation | FLG | Filaggrin | 76 | 79 | 105 | 105 | 89 | 68 | 67 | 59 | 115 | 111 | 91 | 80 |
| | LOR | Loricrin | 235 | 200 | 250 | 196 | 158 | 225 | 193 | 224 | 245 | 231 | 239 | 239 |
| | TCHH | trichohyalin | 140 | 145 | 170 | 145 | 137 | 161 | 127 | 148 | 187 | 156 | 209 | 149 |
| | RPTN | Repetin | 336 | 356 | 456 | 476 | 349 | 403 | 288 | 326 | 402 | 273 | 404 | 307 |
| | CRCT1 | cysteine-rich C-terminal 1 | 160 | 121 | 146 | 148 | 156 | 201 | 175 | 159 | 89 | 172 | 176 | 157 |
| Tight junctions - Adherens junctions | CLDN4 | claudin 4 | 118 | 110 | 132 | 127 | 115 | 147 | 106 | 120 | 111 | 100 | 111 | 122 |
| | CDSN | corneodesmosin | 130 | 181 | 197 | 185 | 146 | 196 | 137 | 150 | 228 | 154 | 160 | 150 |
| | DSC1 | desmocollin 1 | 89 | 121 | 99 | 126 | 86 | 95 | 97 | 114 | 153 | 87 | 99 | 106 |

| mQPA-NHEK-Barrier function and Hydration-32 | Genes | | HAB | | | HAL | | | BBX | | | HABBX | | | HABLX | | |
|---|---|---|---|---|---|---|---|---|---|---|---|---|---|---|---|---|---|
| | | | 0.111% | 0.333% | 1% | 0.041% | 0.123% | 0.37% | 0.122% | 0.367% | 1.1% | 0.111% | 0.333% | 1% | 0.041% | 0.123% | 0.37% |
| | Abbreviation | NCBI data base link | % Control Mean HK | % Control Mean HK | % Control Mean HK | % Control Mean HK | % Control Mean HK | % Control Mean HK | % Control Mean HK | % Control Mean HK | % Control Mean HK | % Control Mean HK | % Control Mean HK | % Control Mean HK | % Control Mean HK | % Control Mean HK | % Control Mean HK |
| Keratinocyte differentiation | FLG | Filaggrin | 63 | 50 | 76 | 102 | 84 | 66 | 154 | 170 | 191 | 98 | 122 | 135 | 117 | 108 | 109 |
| | LOR | Loricrin | 228 | 232 | 249 | 299 | 259 | 231 | 275 | 334 | 549 | 346 | 374 | 641 | 511 | 386 | 496 |
| | TCHH | trichohyalin | 162 | 156 | 173 | 214 | 199 | 178 | 162 | 227 | 339 | 244 | 241 | 371 | 324 | 238 | 308 |
| | RPTN | Repetin | 284 | 394 | 386 | 454 | 330 | 354 | 395 | 408 | 721 | 466 | 611 | 972 | 656 | 603 | 754 |
| | CRCT1 | cysteine-rich C-terminal 1 | 194 | 190 | 225 | 101 | 181 | 159 | 130 | 149 | 152 | 202 | 206 | 207 | 222 | 241 | 232 |
| Tight junctions - Adherens junctions | CLDN4 | claudin 4 | 127 | 130 | 132 | 125 | 129 | 145 | 128 | 122 | 131 | 137 | 130 | 127 | 122 | 135 | 178 |
| | CDSN | corneodesmosin | 145 | 146 | 165 | 71 | 103 | 149 | 187 | 198 | 226 | 87 | 135 | 200 | 373 | 267 | 269 |
| | DSC1 | desmocollin 1 | 117 | 113 | 137 | 98 | 100 | 93 | 199 | 157 | 195 | 135 | 145 | 162 | 130 | 135 | 133 |

**Table 6**. Overview of results from Study 2

| mQPA-NHEK-Barrier function and Hydration-32 | Genes | | HA | | | BL | | | X | | | HABL | | |
|---|---|---|---|---|---|---|---|---|---|---|---|---|---|---|
| | | | 0.014% | 0.041% | 0.123% | 0.014% | 0.041% | 0.123% | 0.014% | 0.041% | 0.123% | 0.014% | 0.041% | 0.123% |
| | Abbreviation | NCBI data base link | % Control Mean HK | % Control Mean HK | % Control Mean HK | % Control Mean HK | % Control Mean HK | % Control Mean HK | % Control Mean HK | % Control Mean HK | % Control Mean HK | % Control Mean HK | % Control Mean HK | % Control Mean HK |
| Inflammation | IL8 | Interleukin 8 | 102 | 66 | 47 | 56 | 71 | 87 | 95 | 87 | 79 | 84 | 79 | 82 |
| Keratinocyte differentiation | FLG | Filaggrin | 121 | 117 | 119 | 188 | 149 | 157 | 111 | 102 | 106 | 207 | 155 | 124 |
| | LOR | Loricrin | 129 | 143 | 121 | 183 | 200 | 172 | 138 | 158 | 172 | 195 | 172 | 170 |
| | TCHH | trichohyalin | 127 | 129 | 123 | 173 | 152 | 159 | 148 | 122 | 130 | 149 | 157 | 138 |
| | RPTN | Repetin | 130 | 146 | 136 | 230 | 184 | 182 | 170 | 137 | 169 | 216 | 190 | 142 |
| Extracellular matrix | HAS3 | Hyaluronan synthase 3 | 177 | 117 | 102 | 128 | 137 | 135 | 128 | 118 | 117 | 129 | 129 | 139 |

| mQPA-NHEK-Barrier function and Hydration-32 | Genes | | HAX | | | BLX | | | HABLX | | |
|---|---|---|---|---|---|---|---|---|---|---|---|
| | | | 0.014% | 0.041% | 0.123% | 0.014% | 0.041% | 0.123% | 0.014% | 0.041% | 0.123% |
| | Abbreviation | NCBI data base link | % Control Mean HK | % Control Mean HK | % Control Mean HK | % Control Mean HK | % Control Mean HK | % Control Mean HK | % Control Mean HK | % Control Mean HK | % Control Mean HK |
| Inflammation | IL8 | Interleukin 8 | 103 | 81 | 56 | 93 | 97 | 92 | 80 | 86 | 88 |
| Keratinocyte differentiation | FLG | Filaggrin | 182 | 200 | 170 | 169 | 157 | 172 | 223 | 191 | 186 |
| | LOR | Loricrin | 274 | 239 | 216 | 247 | 242 | 290 | 295 | 381 | 268 |
| | TCHH | trichohyalin | 175 | 160 | 154 | 181 | 181 | 175 | 193 | 213 | 182 |
| | RPTN | Repetin | 218 | 196 | 192 | 234 | 237 | 262 | 274 | 334 | 241 |
| Extracellular matrix | HAS3 | Hyaluronan synthase 3 | 155 | 112 | 101 | 136 | 136 | 129 | 132 | 173 | 143 |

Up-regulated genes (arbitrary selection for stimulation): % > ≥200; ≥185 <200; >170 <185

Down-regulated genes (arbitrary selection for inhibition): % < 50

## Summary of the results from in vitro studies 1:

**Study 1:** Under the experimental conditions of this study, the compounds (tested at 3 concentrations) all showed an effect on the expression of genes involved in keratinocyte differentiation and epidermal cohesion.

- Compounds HA, X, BB, and BL showed an increase in LOR, RPTN, and CDSN markers.
- Compounds HAB and HAL showed an increase in LOR, TCHH, RPTN, and CRCT1 markers.
- Compounds BBX, HABBX, and HABLX showed a strong increase in LOR, TCHH, RPTN, CRCT1, and also CDSN

markers.

**Study 2:** Under the experimental conditions of this study, the compounds (tested at 3 concentrations and 1 duplicate) all showed an effect on the expression of genes involved mainly in keratinocyte differentiation and epidermal cohesion:

- Compound HA showed a decrease in IL8 marker involved in inflammation
- Compounds BL and X showed a slight increase in the expression of LOR, RPTN, and in a lesser extent TCHH
- Compounds HABL and HAX showed an increase in LOR, RPTN and in a lesser extent TCHH and FLG markers
- Compounds BLX, and HABLX showed a clear increase in LOR, RPTN, TCHH and also FLG markers

Summary of the core data from the two studies showing the synergy effect of expression with the combination of ingredients vs individual ingredients is shown also in **Figure 1.**

Based on the studies, the combination that demonstrates repeatability of the synergy on the expression of LOR and TCHH, and RPTN and variable but relevant expression for CDSN and FLG markers is the combination HABLX. The extent of expression achieved by individual ingredients is slight or even "to be confirmed". The combination, in particular the HABLX blend demonstrates a strong overexpression which was confirmed by two different experiments.

**In vitro studies 2: Evaluation of the HA vs HABLX in loricrin expression on reconstituted human epidermis**

Reconstructed Human Epidermis (RHE) is a lab-grown skin model that mimics real human skin. Scientists create it using cultured human skin cells (keratinocytes) to form a multilayered structure, similar to the natural epidermis. RHEs are used for skin research such as for studying hydration, aging, and diseases, including cosmetic testing to assess product safety without animal testing as well as barrier function studies to understand the skin barrier mechanism, making RHE is a powerful tool for skincare and to demonstrate the potential efficacy of active cosmetic ingredients (Mavon, 2005).

In the present study, the HABLX blend was tested on human epidermis (RHE) to visualize loricrin expression using *in situ* immunolabeling and image analysis. The test conditions were the following, where the "systemic" application refers to the tested substance coming from below in the test model and the "topical" application refers to a situation where the substance is applied on top of the RHE:

- systemic application of both solutions of HA (a blend of 3 hyaluronic acids) and HABLX (triple hyaluronic acid blend + birch leaf extract + xylitol) at 0,041%
- a topical application of serum containing the original HABLX blend (a serum according to Composition Example 2) on the human reconstructed epidermis.

Five-day-old RHE were placed in assay medium and topically treated (5mg/cm$^2$) or not (control) and incubated for 7 days with treatment renewals after 2 and 5 days of incubation. RHEs were frozen in liquid nitrogen and sectioned at 5 $\mu$m thickness for *in situ* immunofluorescent labelling (fluorescent green staining for LOR & red for cell nuclei). The fluorescence intensity obtained was normalized to the total epidermis area (Figure 2). and also quantified in % versus the reference.

In control condition, loricrin is already and primarily present in the granular layer of the normal reconstructed epidermis at the cytoplasmic level of the cells, also present in the stratum corneum.

In the treated condition, with HA, the increase of LOR expression is +2%, and go up +9% when treated with HABLX systemic - it's 4 times increase difference. The serum containing the HABLX, topically applied, shows a stronger increase of +19% of loricrin expression in the reconstructed vs control on a normal (non-stressed/ dehydrated) reconstituted epidermis.

The results are shown also in **Figure 2,** which illustrates loricrin immunostaining and the corresponding fluorescence quantification (%) normalised versus the control.

**In vivo clinical studies 1**

Moisturizing Formulation containing HABLX blend technology and hydration and skin barrier clinical evaluation - test results obtained by using a composition according to Composition example 1 (skin care cream) **Study 1:** Maintenance / reinforcement of the cutaneous barrier and very long-lasting moisturizing effect until 72h

The study was carried with a panel of 22 female volunteers. The effect of the product on the cutaneous barrier immediately (within 30 minutes), twenty-four, forty-eight and seventy-two hours after a single standardized application was analyzed by Transepidermal Water Loss measurements using Tewameter®. The results are shown in **Figure 3,**

Under these study conditions and compared to a non-treated zone, the Moisturizing Formulation (a cream according to Composition Example 1) containing HABLX blend technology presented a cutaneous barrier reinforcement thirty minutes, twenty-four hours and forty-eight hours after its single standardized application on the forearms and a cutaneous barrier maintenance until D3 (t72h): decrease in Transepidermal Water Loss rate of 13% at D0t30’, 9% at D1 (t24h), 10% at D2 (t48h) and 7% at D3 (t72h).

In addition, the moisturizing effect of the product was analyzed immediately, twenty-four, forty-eight and seventy-two hours after a single standardized application using Corneometer®. The results are shown in **Figure 4.**

After a single standardized application on the forearms at the laboratory and in comparison to the non-treated zone, the Moisturizing Formulation containing HABLX blend technology (a cream according to Composition Example 1) presented a significant very long-lasting moisturizing effect thirty minutes, twenty-four, forty-eight and seventy-two hours after its single standardized application: increase in cutaneous hydration rate of 96% at D0t30’, 54% at D1 (t24h), 28% at D2 (t48h) and 18% at D3 (t72h).

**In vivo clinical studies 2**

Operating in a global market, it is important to assess how the combination HABLX would not drive any skin intolerance, knowing there are ethnic variations in skin irritation response. In fact, it is demonstrated that the incidence of adverse skin reaction to cosmetics appears significantly higher in Asian (33.0%) than in Caucasian subjects (11.3%) (Lee et al, 2014). Accordingly, we tested the moisturizing Formulation containing HABLX blend technology on a cohort of Chinese people.

**Study 2:** Cutaneous acceptability evaluation through clinical examination under dermatological control

As Chinese skin is known to be more reactive than Caucasian skin we also tested the capacity of the cream in maintaining human body in good condition as a measure for cutaneous acceptability through clinical examination under dermatological control.

The study was conducted under dermatological control, on 22 healthy Chinese adult subjects, aged from 24 to 50 years old, presenting with normal skin, dry skin, oily skin, combination skin tending to be dry and combination skin tending to be oily on face, in Guangzhou, China.

The results obtained after 14 days of once-daily application of the product demonstrate that the product is very well-tolerated on the cutaneous level. Additional subjective analysis (data not shown) also confirms this. A statistically significant percentage of subjects were satisfied of the product (global appreciation, texture, fragrance, skin hydration, intense hydration, hydration).

**Composition examples**

The following examples illustrate formulations containing the combination of active ingredients (hyaluronic acids, Nordic xylitol and Nordic birch leaf extract and/or Nordic birch bark extract).

**Example 1. Skin care cream, lotion or gel**

| **Ingredients** | **Maximum levels, % (w/w), if present** |
|---|---|
| Oils (e.g. vegetable and/or mineral), waxes and fats (e.g. long chain alcohols) | 60 |
| UV filters | 30 |
| Optional humectants (e.g. glycerine, betaine, saccharide isomerate) | 25 |
| Ethanol (alcohol, alcohol denat.) | 25 |
| Silicones, including volatile silicone alternatives (e.g. alkanes, isododecane) | 20 |
| Emulsifying agents: anionic / amphoteric / non-ionic surfactants | 15 |
| Thickeners | 12 |

(continued)

| Ingredients | Maximum levels, % (w/w), if present |
|---|---|
| Bulking agents | 10 |
| Optional additional ingredients (e.g. vitamins,) | 30 |
| Preservatives, antimicrobials | 2 |
| Colorants | 1 |
| Perfume/ Optional Nordic berry/plant oil, aromatic oil | 1 |
| Hyaluronic acids (e.g. having at least a molecular weight above 3000 kDa, at least one hyaluronic acid having a molecular weight of 200-400 kDa, and at least one hyaluronic acid having a molecular weight of 37-50 kDa) | 0,001%- 50% |
| Nordic Xylitol | 0,001% - 50% |
| Nordic birch leaf or bark extract | 0,0001 - 50% |
| Aqua | to 100 |

**Example 2. Tonic, cosmetic water, serum**

| Ingredients | Maximum levels, % (w/w), if present |
|---|---|
| Ethanol (alcohol, alcohol denat.) | 70 |
| Optional humectants (e.g. glycerine, betaine, saccharide isomerate) | 25 |
| Oils (e.g. vegetable and/or mineral) | 5 |
| Silicones, including volatile silicone alternatives (e.g., alkanes, isododecane) | 5 |
| Bulking agents | 5 |
| Emulsifying agents: anionic / amphoteric / non-ionic surfactants | 5 |
| Thickeners | 2 |
| Optional additional ingredients (e.g. vitamins) | 30 |
| Preservatives, antimicrobials | 2 |
| Perfume/ Optional Nordic berry/plant oil, aromatic oil | 1,5 |
| Hyaluronic acids (e.g. having at least a molecular weight above 3000 kDa, at least one hyaluronic acid having a molecular weight of 200-400 kDa, and at least one hyaluronic acid having a molecular weight of 37-50 kDa) | 0,001%- 50% |
| Nordic Xylitol | 0,001% - 50% |
| Nordic birch leaf or bark extract | 0,0001 - 50% |
| Aqua | to 100 |

As will be understood from the preceding description of the present invention and the illustrative experimental examples, the present invention can be described by reference to the following embodiments:

1. A cosmetic composition comprising, as cosmetically active agents:

- hyaluronic acids;
- Nordic xylitol; and
- Nordic birch leaf extract and/or Nordic birch bark extract.

2. The cosmetic composition according to embodiment 1 comprising, as cosmetically active agents, hyaluronic acids, Nordic xylitol, and Nordic birch leaf extract, the cosmetic composition being a skin care composition.
3. The cosmetic composition according to embodiment 1 comprising, as active agents, hyaluronic acids, Nordic xylitol, and Nordic birch bark extract, the cosmetic composition preferably being a hair and/or scalp care composition.
4. The cosmetic composition according to any one of the preceding embodiments, wherein the hyaluronic acids comprise a blend of hyaluronic acids having different average molecular weights.
5. The cosmetic composition according to any one of the preceding embodiments, wherein the hyaluronic acids comprise a blend of hyaluronic acids having an average molecular weight of about 1000 kDa or more, hyaluronic acids having an average molecular weight of approximately 300 kDa, and hyaluronic acids having an average molecular weight of about 50 kDa.
6. The cosmetic composition according to embodiment 4 or 5, wherein the blend of hyaluronic acids comprises or consists of at least one hyaluronic acid having an average molecular weight above 3000 kDa, at least one hyaluronic acid having an average molecular weight of 200-400 kDa, and at least one hyaluronic acid having an average molecular weight of 37-50 kDa.
7. The cosmetic composition according to any one of the preceding embodiments, wherein the xylitol is Nordic xylitol obtained by processing Nordic oat hulls or Nordic birch wood.
8. The cosmetic composition according to any one of the preceding embodiments, wherein the birch leaf extract and the birch bark extract are water/glycerine extracts obtained from Nordic birch leaves or Nordic birch bark, respectively, in particular from leaves or bark from Nordic birches *Betula pendula, Betula alba* and/or *Betula pubescens.*
9. The cosmetic composition according to any one of the preceding embodiments, wherein the combined concentration of hyaluronic acids, xylitol, and birch leaf extract and/or birch bark extract is 0.001-50%, preferably 0.05-10%, based on the weight of the cosmetic composition, the composition further comprising conventional cosmetically acceptable substances.
10. The cosmetic composition according to any one of the preceding embodiments, wherein the cosmetic composition increases the expression of at least one or more hydration markers selected from the group consisting of loricrin, trichohyalin, repetin, corneodesmosin and filaggrin.
11. The cosmetic composition according to any one of embodiments 1-2 or 4-10, wherein the sole cosmetically active agents of the cosmetic composition consist or consist essentially of hyaluronic acids, Nordic xylitol, and Nordic birch leaf extract, for increasing the expression of skin hydration markers, particularly loricrin and trichohyalin
12. The cosmetic composition according to embodiment 11, wherein the combined concentration of hyaluronic acids, birch leaf extract and xylitol is 0.05-10% by weight of the composition.
13. The cosmetic composition according to any one of embodiments 1 or 3-10, wherein the cosmetic composition is a hair care composition, wherein the sole cosmetically active agents of the cosmetic composition consist of or consist essentially of hyaluronic acids, Nordic xylitol and Nordic birch bark extract, for increasing the expression of trichohyalin.
14. The cosmetic composition according to embodiment 13, wherein the combined concentration of hyaluronic acids, birch bark extract and xylitol is 0.05-10% by weight of the composition.
15. Non-therapeutic, cosmetic use of a composition according to any one of the preceding embodiments for increasing skin hydration and/or skin barrier function on skin of the face, body or the scalp of a human user.
16. Non-therapeutic, cosmetic use of a composition according to any one of embodiments 1 to 14 for upregulating expression of skin hydration markers selected from loricrin, trichohyalin, repetin, corneodesmosin and fillagrin, preferably loricrin and/or trichohyalin.
17. Non-therapeutic, cosmetic use of a combination of hyaluronic acids, Nordic xylitol, and Nordic birch leaf extract and/or Nordic birch bark extract in a cosmetic composition for increasing the skin moisturizing effect and/or the skin barrier strengthening effect of the cosmetic composition on human skin.
18. The use according to embodiment 17, wherein the combined use upregulates or increases the expression of skin hydration markers selected from loricrin, tricohyalin, repetin, corneodesmosin and filaggrin, preferably loricrin and/or trichohyalin.
19. The use according to embodiment 17 or 18 comprising combined use of hyaluronic acids, Nordic xylitol, and Nordic birch leaf extract for increasing the expression of loricrin on human skin.
20. A method for moisturizing skin and for strengthening skin barrier function by upregulating expression of skin hydration markers selected from loricrin, trichohyalin, repetin, corneodesmosin and filaggrin, the method comprising adding hyaluronic acids, Nordic xylitol, Nordic birch leaf extract and/or Nordic birch bark extract into a cosmetic composition and applying the cosmetic composition on the skin of the face, body or the scalp of a user.

While the forgoing examples are illustrative of the principles of the present invention in one or more particular applications, it will be apparent to those of ordinary skill in the art that numerous modifications in form, usage and details of implementation can be made without the exercise of inventive faculty, and without departing from the principles and

concepts of the invention. Accordingly, it is not intended that the invention be limited, except as by the claims set forth below.

The verbs "to comprise" and "to include" are used in this document as open limitations that neither exclude nor require the existence of also un-recited features. The features recited in depending claims are mutually freely combinable unless otherwise explicitly stated. Furthermore, it is to be understood that the use of "a" or "an", that is, a singular form, throughout this document does not exclude a plurality.

INDUSTRIAL APPLICABILITY

At least some embodiments of the present invention find industrial application in cosmetic industry, in particular in the preparation of cosmetic compositions for skin or hair care.

ACRONYMS LIST

| | |
|---|---|
| BB | birch bark extract |
| BBX | birch bark extract + xylitol |
| BL | birch leaf extract |
| CDSN | corneodesmosin |
| CE | cornified cell envelope |
| CLDN1, CLDN4 | Claudin 1, Claudin 4 |
| CRCT1 | Cysteine Rich C-Terminal 1 |
| FLG | filaggrin |
| HA | triple hyaluronic acid blend |
| HAB | triple hyaluronic acid blend + birch bark extract |
| HABBX | triple hyaluronic acid blend + birch bark extract + xylitol |
| HABLX | triple hyaluronic acid blend + birch leaf extract + xylitol |
| HAL | triple hyaluronic acid blend + birch leaf extract |
| HAS3 | hyaluronic acid synthesis marker |
| IRS | inner root sheath |
| LOR | loricrin |
| NHEK | normal human epidermal keratinocytes |
| NMFs | natural moisturizing factors |
| RHE | Reconstituted human epidermis |
| RPTN | repetin |
| RT-qPCR | reverse transcription quantitative polymerase chain reaction |

| | |
|---|---|
| SPRR1A, SPRR1B | markers involved in terminal keratinocyte differentiation |
| TCHH | trichohyalin |
| TGM | transglutaminase 1 |
| TEWL | transepidermal water loss |

CITATION LIST

Patent Literature

Non Patent Literature

M. Armengot-Carbo, Á. Hernández-Martín, A. Torrelo. The Role of Filaggrin in the Skin Barrier and Disease Development. Actas Dermo-Sifiliográficas, Volume 106, Issue 2 2015, Pages 86-95.

Marcel Huber, Georges Siegenthaler, Nicolae Mirancea, Ingo Marenholz, Dean Nizetic, Dirk Breitkreutz, Dietmar Mischke, Daniel Hohl. Isolation and Characterization of Human Repetin, a Member of the Fused Gene Family of the Epidermal Differentiation Complex. Journal of Investigative Dermatology,, Volume 124, (5), 2005, Pages 998-1007

Ishida-Yamamoto A, Igawa S. The biology and regulation of corneodesmosomes. Cell Tissue Res. 2015 Jun;360(3):477-82. doi: 10.1007/s00441-014-2037-z. Epub 2014 Nov 19. PMID: 25407522

Ishitsuka, Y. and Roop, D.R., 2020. Loricrin: Past, present, and future. International Journal of Molecular Sciences, 21(7), p.2271.

E Lee , S Kim, J Lee, S-A Cho, K Shin. Ethnic differences in objective and subjective skin irritation response: an international study. Skin Res Technol ; . 2014 Aug;20(3):265-9

Mavon, A. In Vitro Reconstructed Human Skin and Skin Organ Culture Models Used in Cosmetic Efficacy Testing. Book chapter in Handbook of Cosmetic Science and Technology, 2nd Edition, first edition 2005

Mang Sun 1, et al, Effects of Natural Polyphenols on Skin and Hair Health: A Review, Molecules. 2022 Nov 14;27(22):7832. doi: 10.3390/molecules27227832

Nithya S, Radhika T, Jeddy N., 2015. Loricrin - an overview. J Oral Maxillofac Pathol. 2015 Jan-Apr;19(1):64-8. doi: 10.4103/0973-029X.157204.

Steinert PM, Parry DA, Marekov LN, 2003. Trichohyalin mechanically strengthens the hair follicle: multiple cross-bridging roles in the inner root shealth. The Journal of Biological Chemistry. 278 (42): 41409-19.


## Claims

**1.** A cosmetic composition comprising, as cosmetically active agents:

- hyaluronic acids;
- Nordic xylitol; and
- Nordic birch leaf extract and/or Nordic birch bark extract,

wherein the hyaluronic acids consist of a blend of hyaluronic acids having different average molecular weights.

**2.** The cosmetic composition according to claim 1 comprising, as cosmetically active agents, hyaluronic acids, Nordic xylitol, and Nordic birch leaf extract, the cosmetic composition being a skin care composition.

3. The cosmetic composition according to claim 1 comprising, as active agents, hyaluronic acids, Nordic xylitol, and Nordic birch bark extract, the cosmetic composition preferably being a hair and/or scalp care composition.

4. The cosmetic composition according to any one of the preceding claims, wherein the hyaluronic acids comprise a blend of hyaluronic acids having an average molecular weight of about 1000 kDa or more, hyaluronic acids having an average molecular weight of approximately 300 kDa, and hyaluronic acids having an average molecular weight of about 50 kDa

5. The cosmetic composition according to any one of the preceding claims, wherein the blend of hyaluronic acids comprises or consists of at least one hyaluronic acid having an average molecular weight above 3000 kDa, at least one hyaluronic acid having an average molecular weight of 200-400 kDa, and at least one hyaluronic acid having an average molecular weight of 37-50 kDa.

6. The cosmetic composition according to any one of the preceding claims, wherein the birch leaf extract and the birch bark extract are water/glycerine extracts obtained from Nordic birch leaves or Nordic birch bark, respectively, in particular from leaves or bark from Nordic birches *Betula pendula, Betula alba* and/or *Betula pubescens.*

7. The cosmetic composition according to any one of the preceding claims, wherein the combined concentration of hyaluronic acids, xylitol, and birch leaf extract and/or birch bark extract is 0.001-50%, preferably 0.05-10%, based on the weight of the cosmetic composition, the composition further comprising conventional cosmetically acceptable substances.

8. The cosmetic composition according to any one of the preceding claims, wherein the cosmetic composition increases the expression of at least one or more hydration markers selected from the group consisting of loricrin, trichohyalin, repetin, corneodesmosin and filaggrin.

9. The cosmetic composition according to any one of claims 1-2 or 4-10, wherein the sole cosmetically active agents of the cosmetic composition consist or consist essentially of hyaluronic acids, Nordic xylitol, and Nordic birch leaf extract, for increasing the expression of skin hydration markers selected from loricrin and trichohyalin, and wherein the combined concentration of hyaluronic acids, birch leaf extract and xylitol preferably is 0.05-10% by weight of the composition.

10. The cosmetic composition according to any one of claims 1 or 3-8, wherein the cosmetic composition is a hair care composition, wherein the sole cosmetically active agents of the cosmetic composition consist of or consist essentially of hyaluronic acids, Nordic xylitol and Nordic birch bark extract, for increasing the expression of trichohyalin, and wherein the combined concentration of hyaluronic acids, birch bark extract and xylitol preferably is 0.05-10% by weight of the composition.

11. Non-therapeutic, cosmetic use of a composition according to any one of the preceding claims for increasing skin hydration and/or skin barrier function on skin of the face, body or the scalp of a human user.

12. Non-therapeutic, cosmetic use of a composition according to any one of claims 1 to 10 for upregulating expression of skin hydration markers selected from loricrin, trichohyalin, repetin, corneodesmosin and fillagrin, preferably selected from loricrin and trichohyalin.

13. Non-therapeutic, cosmetic use of a combination of hyaluronic acids, wherein the hyaluronic acids consist of a blend of hyaluronic acids having different average molecular weights, Nordic xylitol, and Nordic birch leaf extract and/or Nordic birch bark extract in a cosmetic composition for increasing the skin moisturizing effect and/or the skin barrier strengthening effect of the cosmetic composition on human skin.

14. The use according to claim 13 wherein the combined use upregulates or increases the expression of skin hydration markers selected from loricrin, tricohyalin, repetin, corneodesmosin and filaggrin, preferably selected from loricrin and trichohyalin.

15. The use according to claim 13 or 14 comprising combined use of hyaluronic acids, Nordic xylitol, and Nordic birch leaf extract for increasing the expression of loricrin on human skin.

16. A method for moisturizing skin and for strengthening skin barrier function by upregulating expression of skin hydration

markers selected from loricrin, trichohyalin, repetin, corneodesmosin and filaggrin, the method comprising adding hyaluronic acids, wherein the hyaluronic acids consist of a blend of hyaluronic acids having different average molecular weights, Nordic xylitol, Nordic birch leaf extract and/or Nordic birch bark extract into a cosmetic composition and applying the cosmetic composition on the skin of the face, body or the scalp of a user.

gene expression % of indivudual ingredients and in combination
( at 0,37% - & 0,041%) % of expression - 100 is the reference /control
500
450
400
350
300
250
200
150
100
HA
BL
X
HABLX
LOR
TCHH
CDSN
LOR
TCHH
FLG

Fig. 1

Loricrin Expression (%) in RHE
125
120
115
110
105
100
95
100
102
109
119
Control
HA (systemic)
HABLX (systemic)
Serum (topical)

Fig. 2

Variations of the Transepidermal Water Loss (in g.m-2.h-1.) after product application
(compared to a non-treated zone)
% variations of Transepidermal Water Loss
0%
-2%
-4%
-6%
-8%
-10%
-12%
-14%
-16%
-18%
-20%
-13%
-9%
-10%
-7%
D0t30'
D1
D2
D3

Fig. 3

Variations of cutaneous hydration rate after product application
(compared to a non-treated zone)
% variations of cutaneous hydration rate
100%
90%
80%
70%
60%
50%
40%
30%
20%
10%
0%
+96%
+54%
+28%
+18%
D0t30'
D1
D2
D3

Fig. 4

Europäisches Patentamt
European Patent Office
Office européen des brevets

# EUROPEAN SEARCH REPORT

Application Number
EP 26 16 6439

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | DATABASE GNPD [Online] "MINTEL"; 12 August 2022 (2022-08-12), "Spray", XP093417937, Database accession no. 9826324 * the whole document * | 1-16 | INV. A61K8/34 A61K8/73 A61K8/9789 A61Q19/00 |
| Y | DATABASE GNPD [Online] "MINTEL"; 15 September 2021 (2021-09-15), "Sliver Birch Soothing Mask", XP093417938, Database accession no. 9005754 * abstract * | 1-16 | |
| Y | WO 2022/135827 A1 (ORIFLAME COSMETICS AG [CH]) 30 June 2022 (2022-06-30) * claim 12 * * example 9 * | 1-16 | TECHNICAL FIELDS SEARCHED (IPC) |
| Y | US 2024/050360 A1 (MOY MELISSA [US] ET AL) 15 February 2024 (2024-02-15) * paragraph [0009] * * paragraph [0081] * * example B * * example 6 * * claim 52 * | 1-16 | A61K A61Q |
| Y | DATABASE GNPD [Online] "MINTEL"; 20 January 2021 (2021-01-20), "Hydrating Ion Serum", XP055935189, Database accession no. 8335453 * the whole document * | 1-16 | |

The present search report has been drawn up for all claims

1

| Place of search | Date of completion of the search | Examiner |
|---|---|---|
| Munich | 22 July 2026 | Hidaoui, Nadia |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

# ANNEX TO THE EUROPEAN SEARCH REPORT ON EUROPEAN PATENT APPLICATION NO. EP 26 16 6439

This annex lists the patent family members relating to the patent documents cited in the above-mentioned European search report. The members are as contained in the European Patent Office EDP file on
The European Patent Office is in no way liable for these particulars which are merely given for the purpose of information.

22-07-2026

| Patent document cited in search report | Publication date | Patent family member(s) | Publication date |
|---|---|---|---|
| WO 2022135827 A1 | 30-06-2022 | CN 116685341 A | 01-09-2023 |
| | | EP 4267103 A1 | 01-11-2023 |
| | | SE 2030376 A1 | 24-06-2022 |
| | | WO 2022135827 A1 | 30-06-2022 |
| US 2024050360 A1 | 15-02-2024 | AU 2023321673 A1 | 06-02-2025 |
| | | CA 3263027 A1 | 15-02-2024 |
| | | EP 4568754 A1 | 18-06-2025 |
| | | US 2024050360 A1 | 15-02-2024 |
| | | WO 2024035908 A1 | 15-02-2024 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **M. ARMENGOT-CARBO** ; **Á. HERNÁNDEZ-MARTÍN** ; **A. TORRELO**. The Role of Filaggrin in the Skin Barrier and Disease Development. *Actas Dermo-Sifiliográficas*, 2015, vol. 106 (2), 86-95
- **MARCEL HUBER** ; **GEORGES SIEGENTHALER** ; **NICOLAE MIRANCEA** ; **INGO MARENHOLZ** ; **DEAN NIZETIC** ; **DIRK BREITKREUTZ** ; **DIETMAR MISCHKE** ; **DANIEL HOHL**. Isolation and Characterization of Human Repetin, a Member of the Fused Gene Family of the Epidermal Differentiation Complex. *Journal of Investigative Dermatology*, 2005, vol. 124 (5), 998-1007
- **ISHIDA-YAMAMOTO A** ; **IGAWA S.** The biology and regulation of corneodesmosomes. *Cell Tissue Res.*, 19 November 2014, vol. 360 (3), 477-82
- **ISHITSUKA, Y.** ; **ROOP, D.R.** Loricrin: Past, present, and future. *International Journal of Molecular Sciences*, 2020, vol. 21 (7), 2271
- **E LEE** ; **S KIM** ; **J LEE** ; **S-A CHO** ; **K SHIN**. Ethnic differences in objective and subjective skin irritation response: an international study. *Skin Res Technol*, August 2014, vol. 20 (3), 265-9
- In Vitro Reconstructed Human Skin and Skin Organ Culture Models Used in Cosmetic Efficacy Testing. **MAVON, A.** Book chapter in Handbook of Cosmetic Science and Technology. 2005
- **MANG SUN 1 et al.** Effects of Natural Polyphenols on Skin and Hair Health: A Review. *Molecules*, 14 November 2022, vol. 27 (22), 7832
- **NITHYA S** ; **RADHIKA T** ; **JEDDY N.** Loricrin - an overview.. *J Oral Maxillofac Pathol.*, January 2015, vol. 19 (1), 64-8
- **STEINERT PM** ; **PARRY DA** ; **MAREKOV LN**. Trichohyalin mechanically strengthens the hair follicle: multiple cross-bridging roles in the inner root shealth. *The Journal of Biological Chemistry*, 2003, vol. 278 (42), 41409-19

**Erroneously filed documents**

**Erroneously filed drawings (or parts thereof)**

gene expression % of indivudual ingredients and in
( at 0,37% - & 0,041%) % of expression - 100 is the re
500
450
400
350
300
250
HA
BL
X

Fig. 1